Europäisches Patentamt

(19) European Patent Office

Office européen des brevets

(11) Publication number: **0 172 841**
**B1**

(12) **EUROPEAN PATENT SPECIFICATION**

(45) Date of publication of patent specification: **16.08.90**

(51) Int. Cl.⁵: **G 01 N 33/487,** G 01 N 21/00

(21) Application number: **85900725.4**

(22) Date of filing: **09.01.85**

(86) International application number:
**PCT/HU85/00001**

(87) International publication number:
**WO 85/03131 18.07.85 Gazette 85/16**

(54) **METHOD FOR DETECTING MALIGN PROLIFERATION.**

(30) Priority: **12.01.84 HU 7884**
**21.03.84 HU 112984**

(43) Date of publication of application:
**05.03.86 Bulletin 86/10**

(45) Publication of the grant of the patent:
**16.08.90 Bulletin 90/33**

(84) Designated Contracting States:
**AT BE CH DE FR GB LI LU NL SE**

(56) References cited:
**EP-A-0 007 214**
**DE-A-2 844 217**

The file contains technical information
submitted after the application was filed and
not included in this specification

(73) Proprietor: **KOVACS, Adam**
**Buza u 2**
**H-2097 Pilisborosjenö (HU)**

(72) Inventor: **KOVACS, Adam**
**Buza u 2**
**H-2097 Pilisborosjenö (HU)**

(74) Representative: **Dipl.-Ing. Schwabe, Dr. Dr.**
**Sandmair, Dr. Marx**
**Stuntzstrasse 16**
**D-8000 München 80 (DE)**

Courier Press, Leamington Spa, England.

**Description**

Technical field

The present invention relates to a diagnostic method for detecting malign proliferation—tumorous diseases, including leukemia—from blood serum, body fluid, cell agglomerates and tissues.

Background art

It is well known that the essential of successful treatment of malign tumorous diseases is the diagnosis thereof in a very early state. Extended research has been carried out recently for developing such diagnostic methods. These methods are generally based on the measurement of the concentration or the change in concentration of certain specific proteins—e.g. enzyme—in the blood. These methods are, however, of controlled area of use, and need longer time and cost. No method has been disclosed so far which would generally be applicable in the diagnosis of tumorous diseases.

Therefore, the present invention aims to provide a simple and quick method for detecting tumorous diseases, possibly in a very eary state thereof.

Disclosure of invention

The present invention is based on the recognition that in the samples of blood serum, body fluids, cell agglomerates and tissue preparations prepared suitably under physiological conditions, characteristic protein filaments can be observed by optical methods. These filaments are characterized by the so-called "terminal effect", i.e. in microscopic picture the ends of the said filaments in every case reach or advance in the possible shortest way towards a point of another filament provided that the sample originates from a healthy organ. If the same has been obtained from a tumorous organ, the "terminal effect" is not characteristic, i.e. the filament system characteristic to the healthy state does not occur at all or the filaments meet each other partly only.

The characteristic features of the terminal effect mentioned above and the formation thereof is explained herebelow in the example of blood serum. It is obvious, however, that by making preparations from samples of other body fluids, cell agglomerates or tissues, the appearance of terminal effect will be alike.

Brief descrpiption of drawings

In the followings the invention is demonstrated by the figures more in details.

Fig. 1 is a photograph of dried blood serum taken by electron microscope.

Fig. 2 is a photograph of blood serum preparations of healthy patient taken by electron microscope.

Fig. 3 is the photograph of blood serum preparation of tumorous patient taken by electron microscope.

Fig. 4 is a photograph of the filament structure of blood serum preparion from a healthy patient.

Fig. 5 is the photograph of the filament structure of blood serum preparation from a tumorous patient.

Fig. 6 is a section of the photograph of blood serum preparation from healthy patient showing the terminal effect.

Fig. 7 is the photograph of ascites fluid preparation from a healthy patient.

Fig. 8 is the photograph of ascites fluid preparation obtained from a tumorous organ.

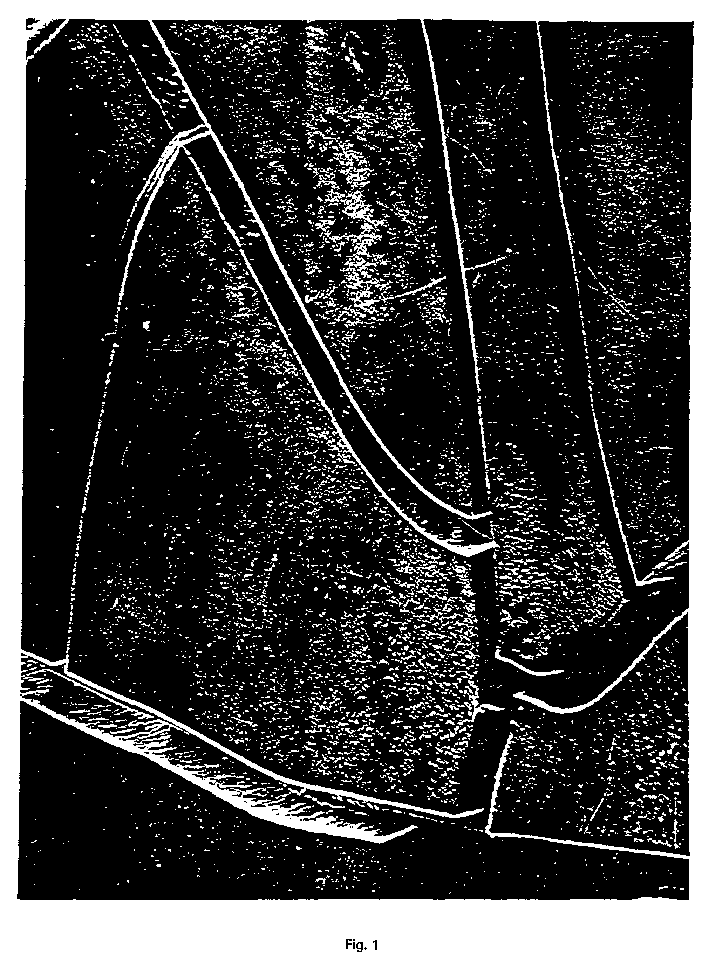

When blood taken venally is allowed to sediment and then the serum is dropped on microscope slide, a characteristic picture is obtained after drying under mild conditions. Figure 1 shows interesting formations, filaments in the crackled preparation.

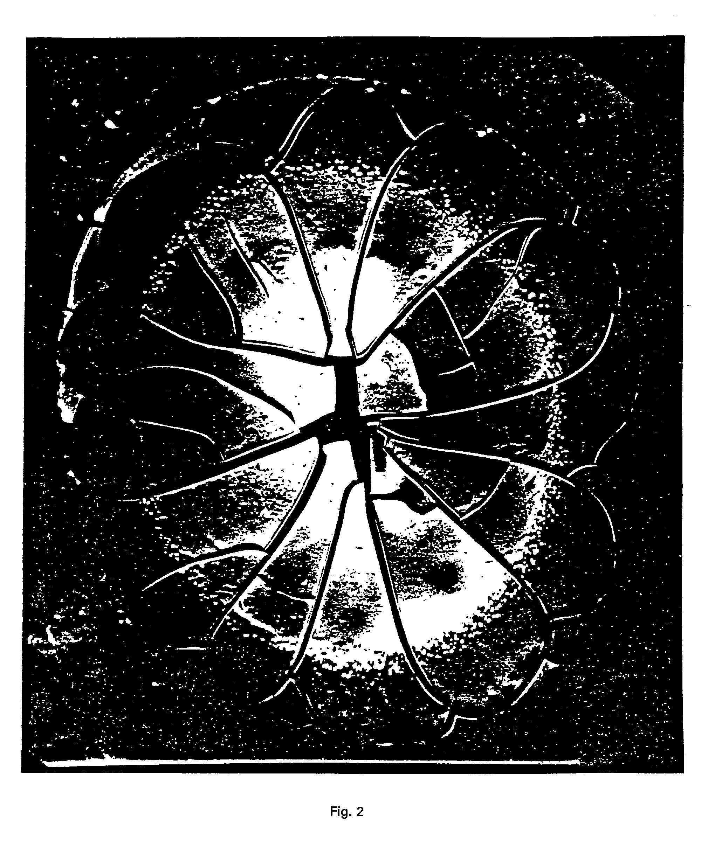

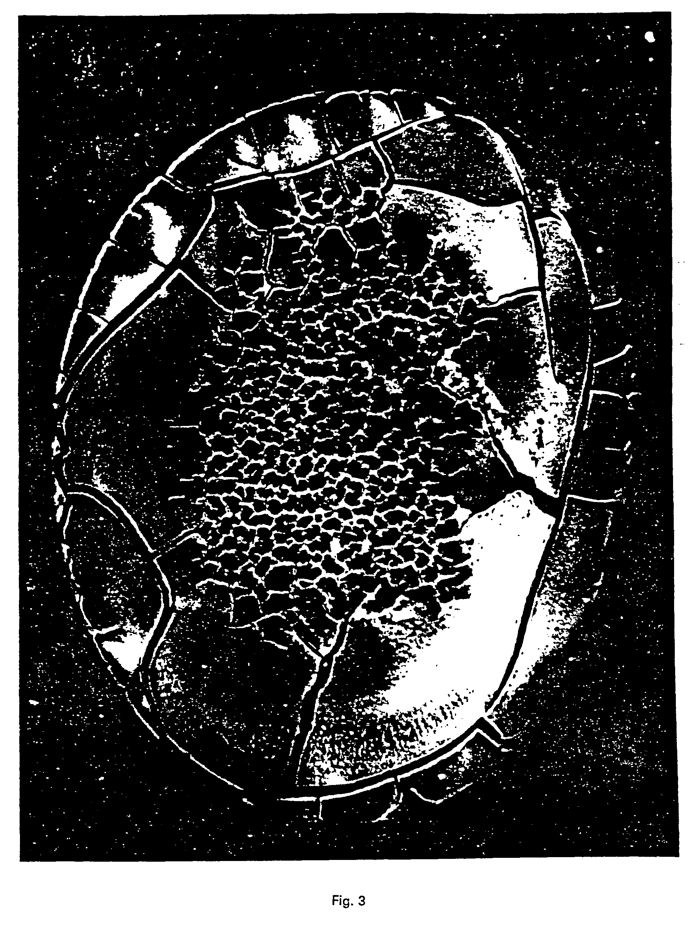

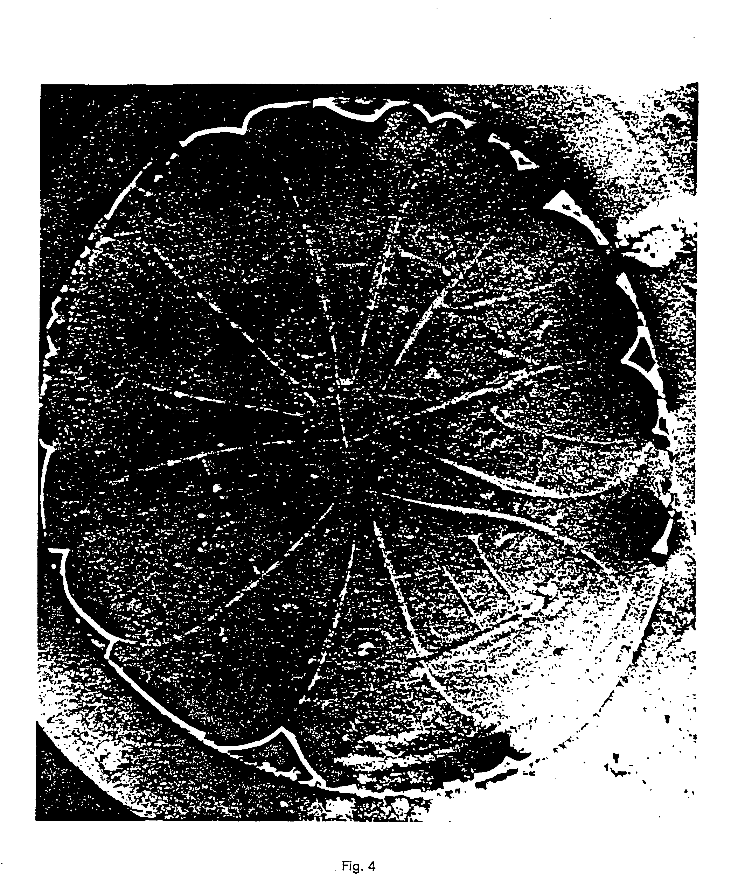

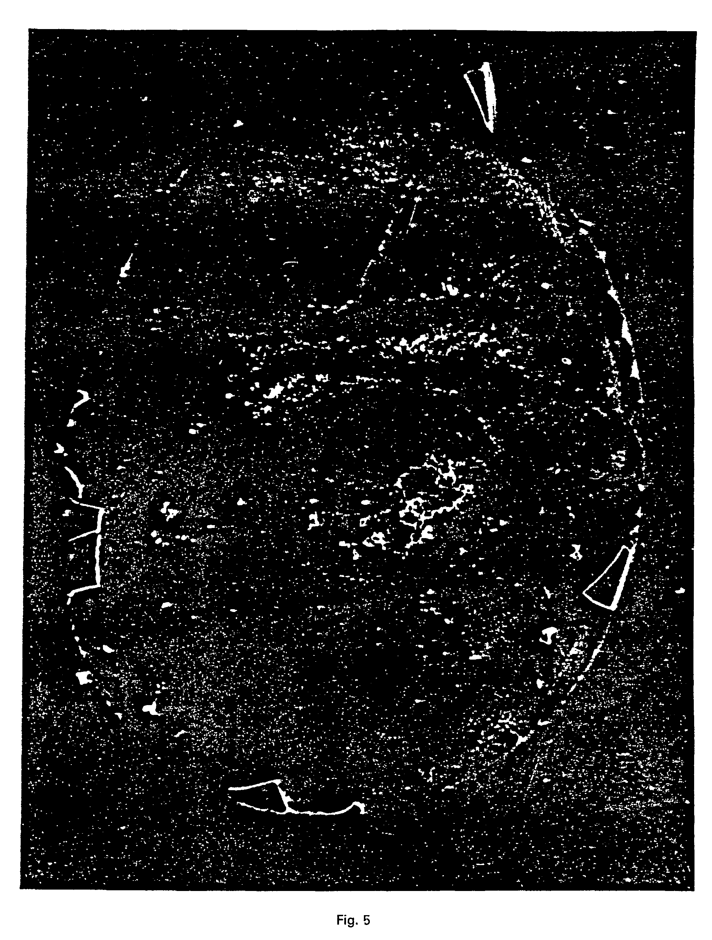

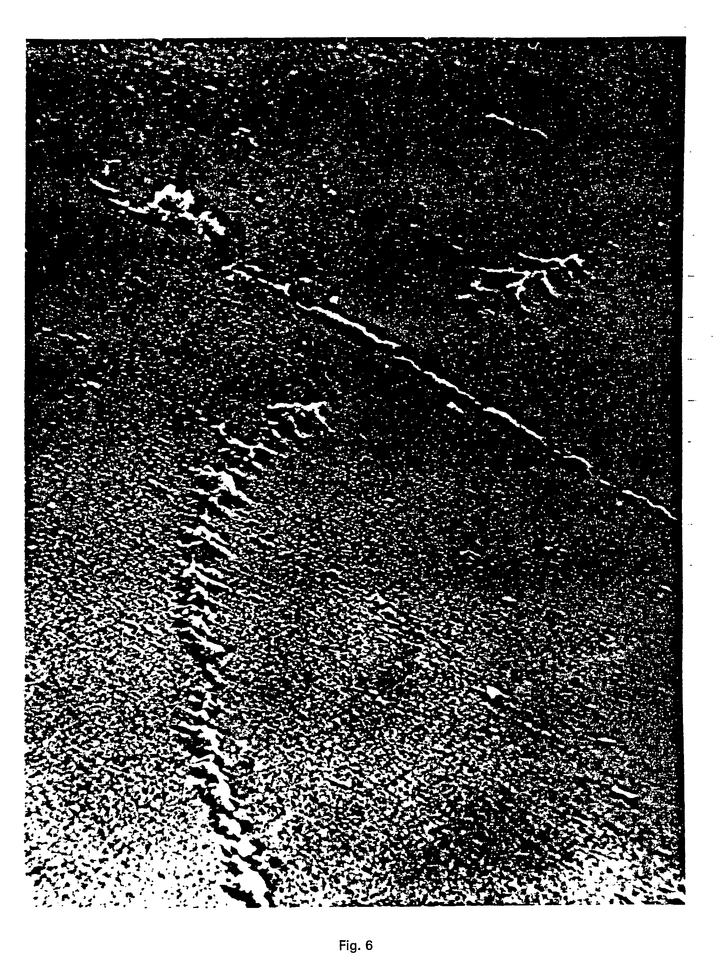

It has been found that the picture of blood serums from healthy and tumorous organism, respectively, are basically different after drying. Fig. 2 shows the dried blood serum preparation of a healthy patient while Fig. 3 shows the same from a patient suffering from malign tumour. The splits shown on the photographs always form along the filaments; when the upper layer of the preparation is removed, the filaments structures according to Figs. 4 and 5 are obtained. Fig. 4 shows clearly that the filaments are in close contact with each other, i.e. the terminal effect, shown in Fig. 6 more in details, occurred. Fig. 5 shows no terminal effect, neither filaments were formed; the structure refers unambiguously to the presence of malign processes.

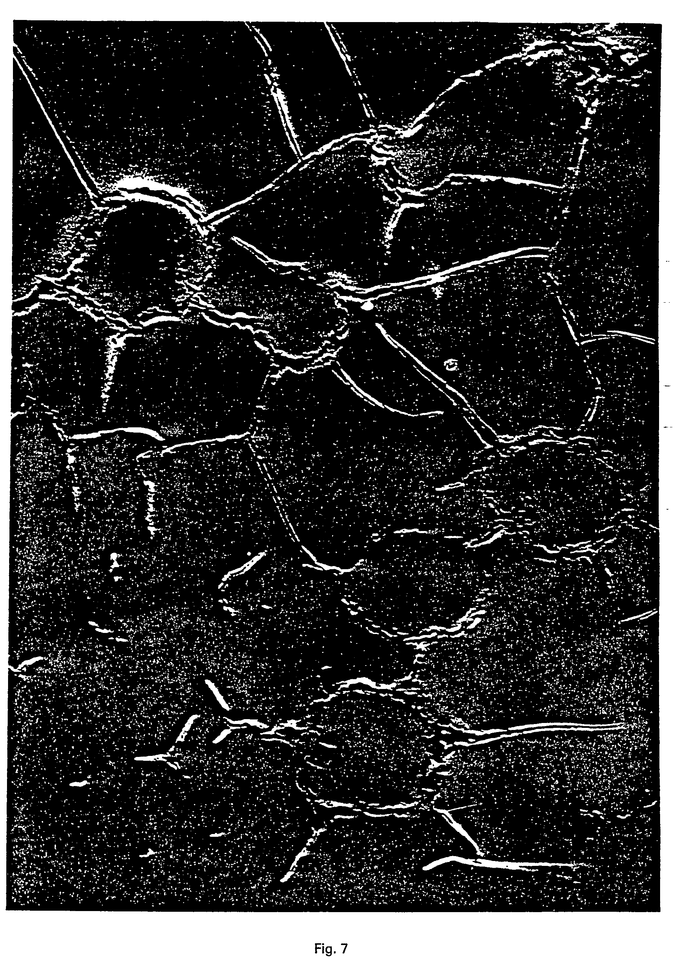

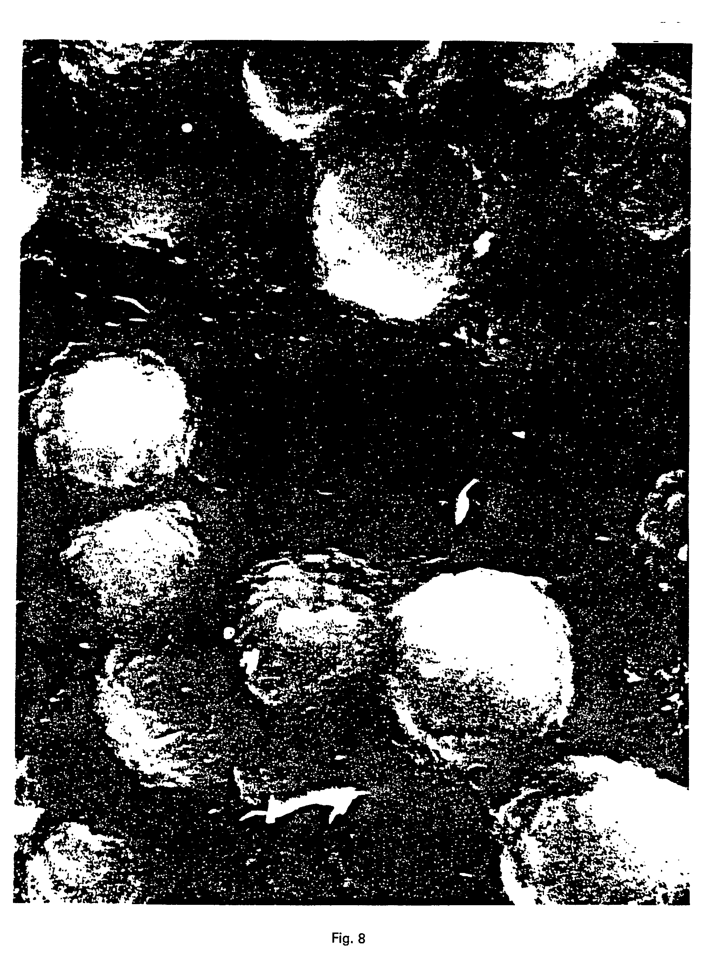

Similar pictures may be obtained when examining the samples of ascites fluids instead of blood serum samples. Figs. 7 and 8 show the picture of these preparation obtained from healthy and tumorous organism, respectively.

It can clearly be seen from Fig. 7 that in case of healthy cells intercellular contact was formed by the filaments and the characteristic splits occurred along these. Fig. 8 shows the lack of terminal effect, i.e. the presence of malign processes in the organism.

The method according to the invention was worked out on basis of our aforesaid experiences. The method comprises optically examining blood serum, body fluid, cell agglomerate or tissue preparations having been dried preferably under physiological conditions and the pathological change is proved on basis of at least partial lack of the terminal effect.

The samples can be obtained generally by methods known per se. It is preferred, however, to prepare them under controlled conditions as the method can be standardised by this way and the different samples can be compared easier.

In case of examining blood samples, the preferred process is as follows:

Native blood is taken, the blood serum is prepared suitably by sedimenting or centrifuging, then the serum is dropped on a suitable support. Good preparations can be obtained when applying a drop of the volume of 1 to 20 µl or about the same amount is distributed on the microscope slide.

Drying of the fluid is generally carried out at controlled temperature, preferably at 20 to 50°C by maintaining constant moisture content and pressure. The drying can be acocmplished by using vacuum, however, it is preferred to work at atmospheric pressure. Drying may also be performed by freezing to the critical point when the water in the sample separates in microcrystalline form and the splits will again follow the filaments.

Examination and evaluation of the samples are made on basix of the terminal effect. The organism can be regarded as healthy if the terminal effect can be found in the whole of the sample. If the terminal effect is lacking—even if only in part—, the phenomenon refers to malign process.

Evaluation is accomplished visually as the most simple way, however, it is also possible to use special apparatus and methods, respectively, which might ease the identification. Processes like these include Urbancsek-algorhythm method, Fourier-transformation algorhythm, the method of closed graphs, counting the black/white/black or reversed transitions or any combination thereof. However, it is to be understood that the mode of evaluation is not limited to the above anyway and the choice of the evaluation method does not form a part of the scope of protection.

The diagnostic method according to the invention was controlled in tests carried out on more than 1000 patients and was proved to be effective with a very good accuracy in determining tumorous states of different type. The method is very advantageous as it is very quick, thus it can be used for screenings, for following therapies, as well as in the search for anti-tumour compounds for quick in vitro screening of the effect of test materials.

Example

A group consisting of 1164 patients was tested with the method according to the invention. During the tests the results obtained from the pictures of dried blood serum were compared with clinical histological diagnoses. When evaluating, three sub-groups were formed as shown in Table II.

The first group consisted of patients which were proved to have malign tumour according to histological examination. The method according to the invention /MTA/ showed malignity in 358 of the 383 cases (94%). Table I herebelow shows the distribution of different malign diseases within this group.

TABLE I

| Malign disease | Number of cases |
|---|---|
| Leukemia | 74 |
| Malignant lymphomas | 93 |
| Head and neck cancer | 9 |
| Lung cancer | 17 |
| Malignant breast neoplasms | 41 |
| Malignant neoplasm of digestive organs | 48 |
| Malignant neoplasm of female genital organs | 12 |
| Malignant neoplasm of male genital organs | 56 |
| Brain tumour | 7 |
| Neoplasm of urinary system | 20 |
| Epithelioma | 6 |

The second group served as control, consisting of healthy patients below an age of 25. The method according to the invention found 230 from the 242 of the group to be healthy (95%).

The third group consisted of patients of an age between 25 and 85 who did not suffer from tumorous disease according to histological examination but had other illness, such as hepatitis, pneumonia, hypertonia, diabetes, benign tumour, etc. The MTA proved 376 cases as negative from the total of 539 (70%). As mentioned, at this group clinical diagnosis has not detected malign tumorous disease but the possibility could not be eliminated.

A summary of the above test is shown in Table II herebelow.

TABLE II

| Groups | Total of patients | MTA + | MTA − | MTA total% |
|---|---|---|---|---|
| Histologically proved tumorous patients | 383 | 358 | 25 | 94 |
| Healthy control groups (under the age of 25) | 242 | 12 | 230 | 95 |
| Mixed group (no clinically detected cancer but possibility cannot be eliminated). | 539 | 163 | 376 | 70 |

It is believed on basis of the results obtained at the first two groups of MTA detects the malign processes in their very early state, especially when there are no serious symptoms and malignant tumour cannot be detected or only in certain cases by other clinical methods. From the patients of the "mixed group" proved to be positive by the method according to the present invention 7 were later proved to have malign tumour by subsequent clinical examinations while the others were kept under additional observation.

**Claims**

1. A method for detecting malign proliferation characterized in that a sample of blood serum, body fluid, cell agglomerates or tissue is dried under mild physiological conditions on a suitable optical slide and the sample thus obtained is examined by light or electron microscopy and the pathological change is proved on basis of at least a partial retraction of characteristic protein filaments which in the healthy state reach or advance each other in the possible shortest way towards a point of another filament.
2. The method according to Claim 1 wherein the sample is blood serum.
3. The method according to Claim 1 wherein the sample is ascites fluid.

**Patentansprüche**

1. Verfahren zum Nachweis maligner Proliferation, dadurch gekennzeichnet, daß eine Probe Blutserum, Körperflüssigkeit, Zellagglomerate oder Gewebe unter milden physiologischen Bedingungen auf einem geeigneten optischen Träger getrocknet wird, und die so erhaltene Probe mit Hilfe von Licht- oder Elektronenmikroskopie untersucht wird, und die pathologische Veränderung auf der Grundlage wenigstens einer teilweisen Retraktion charakteristischer Proteinfasern geprüft wird, die im gesunden Zustand zueinander reichen oder sich auf dem kürzestmöglichen Wege aufeinander auf einen Punkt einer anderen Faser hin zubewegen.
2. Verfahren nach Anspruch 1, worin die Probe Blutserum ist.
3. Verfahren nach Anspruch 1, worin die Probe Ascitesflüssigkeit ist.

**Revendications**

1. Procédé de dépistage de la prolifération des tumeurs malignes, caractérisé en ce qu'on sèche un échantillon de sérum sanguin, d'un liquide corporel, d'agglomérats de cellules ou de tissus dans des conditions physiologiques modérées sur une lamelle optique appropriée, l'on examine l'échantillon ainsi obtenu à la lumière ou au microscope électronique et l'on prouve le changement pathologique sur la base d'au moins une rétraction partielle des filaments protéiques caractéristiques qui, à l'état sain, atteignent ou se précèdent mutuellement de la manière la plus courte possible vers un point d'un autre filament.
2. Procédé selon la revendication 1, caractérisé en ce que l'échantillon est le sérum sanguin.
3. Procédé selon la revendication 1, caractérisé en ce que l'échantillon est un liquide ascitique.

EP 0 172 841 B1

Fig. 1

Fig. 2

Fig. 3

Fig. 4

Fig. 5

Fig. 6

Fig. 7

Fig. 8